# EUROPEAN PATENT APPLICATION

(11) **EP 4 344 641 A1**
(43) Date of publication of application: **03.04.2024**
(21) Application number: 22198618.5
(22) Date of filing: 29.09.2022
(51) Int. Cl.: A61B 6/00, H02G 3/38, A61B 6/03

(54) **CABLE GUIDANCE IN MOVABLE MEDICAL APPLIANCES**

(71) Applicant: Koninklijke Philips N.V., 5656 AG Eindhoven (NL)
(72) Inventor: KROON, Gerardus Bernardus, Eindhoven (NL)
(74) Representative: Philips Intellectual Property & Standards

(57) **Abstract**

The present invention relates to providing cable guidance in an at least partly moving medical appliance. In order to provide a cable connection with less impact on the usability of the medical facility, a cable guidance (10) with a feedthrough movable along a guide rail is provided. The cable guidance comprises a cable duct (12) and a cover arrangement (14). The cable duct is extending along a longitudinal direction (*D_{L}*) and comprises a cable receiving and guiding space (16). The cable duct is configured to accommodate a cable connection comprising a number of cables. The cover arrangement comprises a floor section (18) for permanently delimiting the cable duct and for forming a floor part (20). A support mechanism (22) is provided comprising a longitudinal support element (24) that is configured to support a cantilevering edge of the floor section in the region of the cable duct. The support element is configured to be partly temporarily displaced to provide a movable passageway (26) from the cable duct to a connection opening.

## Description

### FIELD OF THE INVENTION

The present invention relates to providing cables in an at least partly moving medical appliance, i.e. to cable guidance in movable medical appliances. The present invention relates in particular to a cable guidance with a feedthrough movable along a guide rail, to a medical system and to a method for cable guidance.

### BACKGROUND OF THE INVENTION

Medical interventions and procedures may involve the use of medical appliances, which can be rather heavy and which may be provided in a fixed manner within a medical facility. An example for a medical appliance used for imaging is a CT-scanner, which can be heavy and bulky. In order to be able to perform medical interventions and imaging, a movable subject support, also known as patient table, may be arranged. As an example, the subject, also referred to as patient, is moved in and out the bore of a CT-scanner to make an X-ray imaging scan. However, in some clinical procedures, it would be an advantage not to move the patient, but the CT-scanner. This may be provided by placing the CT-scanner on rails to allow a linear movement. The supply of the CT-scanner, e.g. with electric energy and also with control signals, is provided by cable connections. The cable connection in the floor area may be an obstacle within the medical facility. Some solutions provide a cable connection via the ceiling, but it has been shown that this may interfere with air supply and other systems arranged within the ceiling area of the facility. As an example, US 2014/037071 A1 describes a cable canal in the floor section with a guide channel having a slot covered with a telescoping cover that adapts to the travel movement of the gantry. However, it has been shown that guide channels may be too narrow to provide sufficient space for the cables to be accommodated.

### SUMMARY OF THE INVENTION

There may thus be a need to provide a cable connection with improved usability within the medical facility.

The object of the present invention is solved by the subject-matter of the independent claims; further embodiments are incorporated in the dependent claims. It should be noted that the following described aspects of the invention apply also for the cable guidance with a feedthrough movable along a guide rail, for the medical system and for the method for cable guidance.

According to the present invention, a cable guidance with a feedthrough movable along a guide rail is provided. The cable guidance comprises a cable duct and a cover arrangement. The cable duct is extending along a longitudinal direction and comprises a cable receiving and guiding space. The cable duct is configured to accommodate a cable connection comprising a number of cables. The cover arrangement comprises a floor section for permanently delimiting the cable duct and for forming a floor part. A support mechanism is provided comprising a longitudinal support element that is configured to support a cantilevering edge of the floor section in the region of the cable duct. The support element is configured to be partly temporarily displaced to provide a movable passageway from the cable duct to a connection opening.

As an effect, the flexible cables can be arranged in ducts below the floor surface, which ducts can be provided as wide troughs in which the cables can move freely. To be able to walk and stand everywhere, this trough is covered by e.g. steel plates. These plates normally deflect when a load is on top, like people, moveable equipment or the like. However, this deflection should not hamper the movement of the flexible cable duct underneath. By providing the support mechanism that supports the floor section but that also allows the cable feedthrough, a floor connection is provided with improved characteristics.

According to an example, the support element comprises a longitudinal flat member that comprises a stiffness transverse to the longitudinal direction in a plane direction of the flat member. The support element is deformable in a direction transverse to the longitudinal direction and transverse to the plane direction.

As an advantage, the support element can be moved (horizontally) between the two positions while being stable in the (vertical) load bearing direction.

In an option, the support element is provided as a toothed belt structure.

Such structures are available and have proven long-term stability. Instead of transferring pulling, i.e. tensile forces, the toothed belt structures are subject to a transverse load, not intended by their original use though. The toothed belt structure, "originally" intended to provide a temporal engaging with a toothed wheel or the like, provides improvement of stability in the transverse direction. The plurality of "ribs" of the toothed belt structure provide stability in the transverse direction of the toothed belt.

In a further example, the support element is provided as chain-like structure.

According to an example, a displacement mechanism is provided that is attachable to a movable medical equipment to which cables are supplied. The displacement mechanism comprises guiding elements which are configured to temporarily guide the support mechanism from a supporting position to a non-supporting position.

The displacement mechanism provides a displacement only for a predetermined section of the support element. The further, major portion still acts as support.

According to an example, the guiding elements comprise a first pair of rotating guides and a second pair of rotating guides. The support element is arranged between the rotating guides of each pair. The passageway is provided between the first pair of rotating guides and the second pair of rotating guides.

According to an example, the support mechanism comprises a top strip arranged for supporting the floor. The support element is provided underneath the top strip for holding the top strip. According to an example, the support mechanism also comprises a bottom strip. The top strip comprises a top abutting surface and the bottom strip comprises a bottom abutting surface. The support element is clamped between the top abutting surface and the bottom abutting surface when supporting the floor. At least one of the top strip and the bottom strip comprises a tapered edge for facilitating the insertion of the support element between the top and bottom abutting surfaces.

This facilitates the displacement of the support element. The strips can also act as abutting surface to have a defined seat for the support element in the supporting position.

According to an example, the displacement mechanism comprises at least one floor lifting element configured to lift a part of the floor section where the support element is currently being displaced.

Lifting the floor provides less friction which facilitates the displacement of the support element.

According to an example, the cable duct comprises a lateral part that is open on an upper side; and the cover arrangement also comprises a longitudinal cover section for temporal closing the upper side of the lateral part. Further, a lifting mechanism is provided that is configured to temporarily lift a part of the cover section such that a temporal connection opening of the cable duct is provided for a feedthrough of a cable bundle.

According to an example, the cover section comprises a stiffness transverse to the longitudinal direction in a spanning direction across the cable duct. Further, the cover section is deformable in a direction transverse to the longitudinal direction and transverse to the spanning direction.

According to the present invention, also a medical system is provided. The system comprises a medical appliance, a movable support, a cable supply for a cable connection of the medical appliance and a cable guidance according to one of the preceding examples. The cable supply comprises a plurality of cables. The medical appliance is movably supported by the movable support. Further, the plurality of cables is arranged with the cable duct of the cable guidance and extending through the passageway and the connection opening to the medical appliance.

According to an example, the movable support comprises at least one rail track with at least one linear bearing.

According to an example, the medical appliance is a CT scanner.

According to the present invention, also a method for cable guidance is provided. The method comprises the following steps:
- providing a longitudinal support element for supporting a cantilevering edge of the floor section in the region of the cable duct;
- temporarily displacing the support element to provide a passageway from the cable duct to a connection opening; and
- guiding a cable connection through the connection opening.

According to an aspect, a moving or movable opening is provided for the cable guidance. The support of the floor plates is provided in an adaptive manner. The moving opening is achieved by providing an enclosing segment that allows a partial displacement to form a local opening for the cable guidance.

According to an aspect, the concept provides floor plates supported by the floor at one side, and by a stationary support belt at the other side. The belt is pushed away to allow the cables to enter the gantry. The feed of cables is at the side of the gantry. A support chain for the cover can be provided stationary.

According to an aspect, moveable gap support floor plates are provided. The gantry cables need to go through the moveable gap support to get from the cable chains in the cable duct into the carriage. Since the carriage moves back and forth, the gap in the moveable gap support moves with the gantry.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a cable guidance with a feedthrough movable along a guide rail. A schematic top view is shown in the upper half of the drawing. A schematic vertical cross-section is shown in the lower half of the drawing. A first cross-section (upper cross-section) shows a supporting position, a second cross-section (lower cross-section) shows a non-supporting position in which the cable feedthrough is achieved.
Fig. 2 schematically shows an example of a medical system comprising an at least partly movable medical appliance a cable supply for a cable connection of the medical appliance and a cable guidance. A schematic top view is shown in the upper half; a schematic vertical cross-section is shown in the lower half.
Fig. 3 shows basic steps of an example of a method for cable guidance.
Fig. 4 shows a perspective view of a part of a movable gantry of a further example of a medical system.
Fig. 5 shows another example of the cable guidance in a perspective view.
Fig. 6 shows a detail of the cable guidance of Fig. 5.
Fig. 7 shows a perspective bottom view of the detail of Fig. 6.
Fig. 8 shows a transverse cross-section of another example of a cable guidance.
Fig. 9 shows a perspective view of the example of Fig. 8.
Fig. 10 shows a top view of the example of Fig. 8 and Fig. 9.
Fig. 11 shows a perspective view of the example of Fig. 10 with partly not-shown floor panels.
Fig. 12 shows further perspective view of the example of Fig. 10 with intact floor panels.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

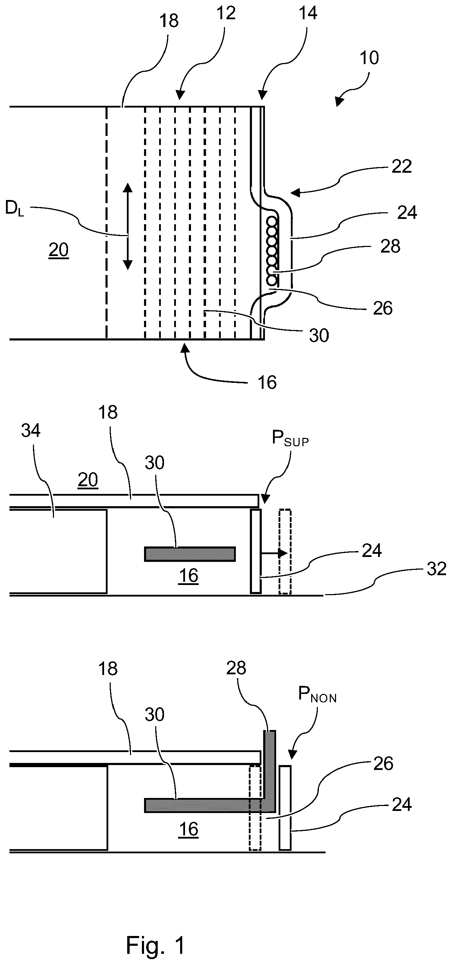

Fig. 1 schematically shows an example of a cable guidance 10 with a feedthrough movable along a guide rail. A schematic top view is shown in the upper half of the drawing. A schematic vertical cross-section is shown in the lower half of the drawing. A first cross-section (upper cross-section) shows a supporting position, a second cross-section (lower cross-section) shows a non-supporting position in which the cable feedthrough is achieved.

The cable guidance 10 comprises a cable duct 12 and a cover arrangement 14. The cable duct 12 is extending along a longitudinal direction *D_{L}* and comprises a cable receiving and guiding space 16. The cable duct 12 is configured to accommodate a cable connection comprising a number of cables. The cover arrangement 14 comprises a floor section 18 for permanently delimiting the cable duct 12 and for forming a floor part 20. A support mechanism 22 is provided comprising a longitudinal support element 24 that is configured to support a cantilevering edge of the floor section in the region of the cable duct 12. Further, the support element 24 is configured to be partly temporarily displaced to provide a movable passageway 26 from the cable duct 12 to a connection opening (see also below).

In Fig. 1, cables 28 are indicated as being guided through the passageway 26. Further, a cable bundle 30 is schematically shown being arranged within the cable receiving and guiding space 16.

A floor base construction is indicated with a line 32. A further floor base 34 is also indicated. For example, the cable receiving and guiding space 16 is arranged as a recess within the further floor base 34.

The term "cable guidance" relates to providing a possible routing for a cable connection. In a simple form, a cable guidance is a cable canal, or cable duct, which provides a space to arrange the cable(s). In the context of the present invention, the cable guidance comprises fixed sections, i.e. sections where the cables are not further moved once installed, but also movable sections, i.e. sections where the cables are moved e.g. during use of a machine or equipment.

The term "feedthrough" relates to a free passageway for the cables. The term expresses that the cable is arranged, i.e. guided, from a canal type part to another part, in this case to the medical appliance housing or base.

The term "cable duct" relates to a longitudinal space provided to arrange the cables within. The cables are then accommodated in the duct. The duct itself is just the (empty) space. It can be formed by cable canal element defining the space, but it can also be provided by arranging a respectively formed recess within an existing structure like a duct formed within a concrete floor slab.

The term "cover arrangement" relates to components and elements that enclose the cable duct at least on one side. As an option, the cover arrangement can provide an upper enclosing surface. As another option, in addition or alternatively, the cover arrangement can provide a lateral enclosing surface.

The term "longitudinal direction" relates to a general direction along which e.g. the movement of an appliance takes place. The longitudinal direction can be a linear direction, or may be curved or angled or a combination thereof.

The term "cable receiving and guiding space" relates to the provided space in which the cables can be arranged. The cable receiving and guiding space is the designed space for the cables, e.g. as a cable canal.

The term "floor section" relates to a part of the floor of the room in a medical facility. In an example, the floor section comprises several floor elements. In another example, the floor section comprises a continuous floor element.

The term "support mechanism" relates to a support of the floor section, e.g. the floor panels. The support is provided with moving parts to change the support during a movement of a device along the longitudinal direction.

The term "displaced" relates to moving a part or region of the support element from a supporting position to a non-supporting, but opening position. In the displaced position, the part or region of the support element is moved to the side for a predetermined distance large enough to allow the guidance of the cables.

The support element can also be referred to as support section.

The passageway can also be referred to as cable exit or cable feedthrough.
As an example for a field of use, the invention as proposed can be used for applications where a moving opening in the floor needs to be created. For example, it is used for CT system on rails.

In an example, the cable may be provided with about 20 to 30 lines.

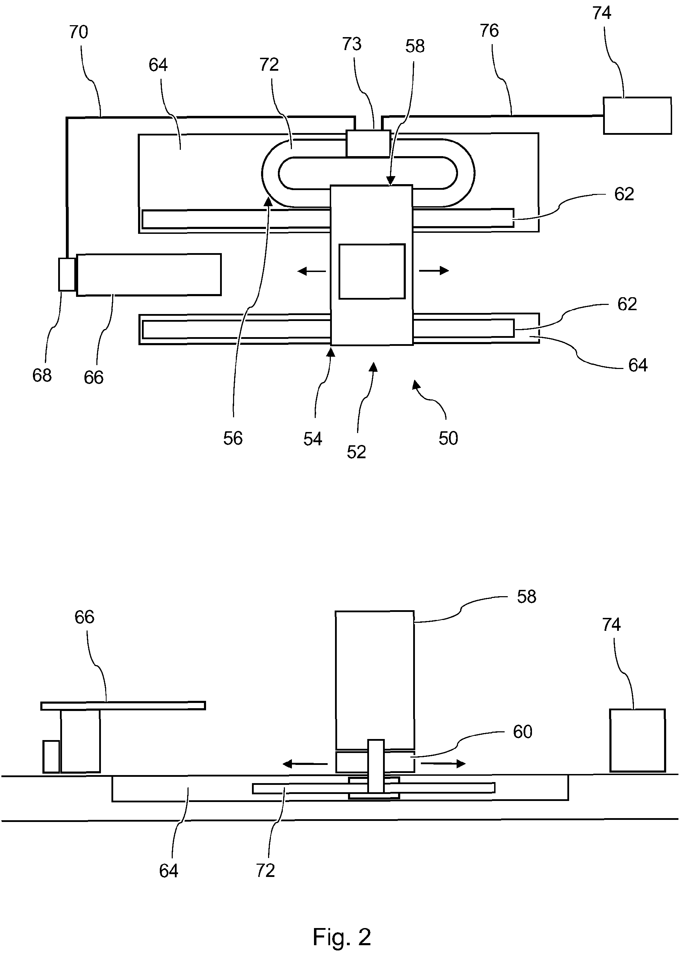

Fig. 2 schematically shows an example of a medical system 50 comprising an at least partly movable medical appliance 52 with a cable supply for a cable connection of the medical appliance and a cable guidance. A schematic top view is shown in the upper half; a schematic vertical cross-section is shown in the lower half.

The medical system 50 comprises the medical appliance 52, a movable support 54. The medical system 50 further comprises a cable supply 56 for a cable connection of the medical appliance 52. The medical system 50 also comprises an example of the cable guidance 10 according to one of the preceding and following examples. The cable supply 56 comprises a plurality of cables. The medical appliance i52 s movably supported by the movable support 54. The plurality of cables is arranged within the cable duct 12 of the cable guidance 10 and is extending through the passageway and the connection opening to the medical appliance 52.

As an option, the medical appliance 52 is provided as a CT system 58 comprising a carriage 60, supported movably on rail tracks 62. A floor structure 64 may be provided in addition. The carriage acts as a mount for a gantry (not shown). A subject support 66, also referred to as patient table, is arranged such that the gantry can be moved in relation to the subject support 66 for imaging e.g. a subject on the subject support 66. As an option, a peripheral devices connection box 68 is provided on one of the front ends of the subject support 66. Further, a connection 70 is indicated to be connected with a cable chain assembly 72. A connection interface 73 is shown. Still further, a CT support 74 is indicated for supplying the CT with electric energy. The CT support 74 is connected with the cable chain assembly 72 via static gantry cables 76. The cable chain assembly 72 provides a loop like arrangement of the plurality of cables arranged within a cable receiving portion, i.e. a recess in the floor structure.

The term "medical appliance" relates to equipment used in medical facilities. As an example for the equipment, the medical appliance is an imaging system, like an X-ray system. For example, a CT scanner is provided with a gantry. In another example, a movable C-arm X-ray system is provided. In further examples, the medical appliance comprises other imaging modalities like MRI or PET imaging systems. In a further example, the medical appliance is an operating robot. In a still further example, the medical appliance is a patient support system like respiratory or cardiac support devices. The term "movable support" relates to a base that allows to move the medical appliance, e.g. along a determined rail or track

The term "cable supply" relates to one or more cables provided for connection of the appliance. The cables are provided for energy supply and/or data transmission, e.g. control signals and data generated with the appliance.

As an option, the movable support 54 comprises at least one rail track with at least one linear bearing. As indicated above, in an option, the medical appliance is a CT scanner.

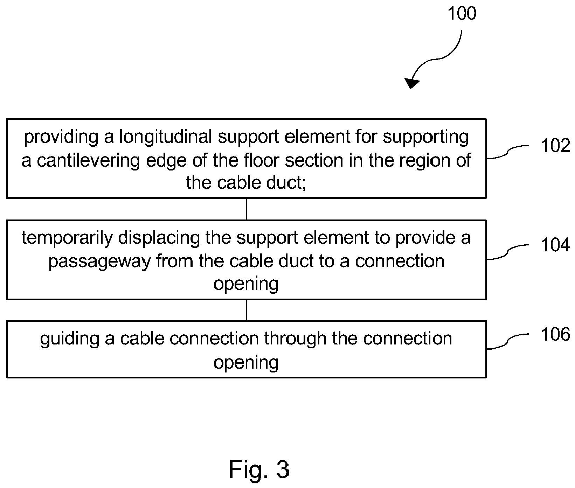

Fig. 3 shows basic steps of an example of a method 100 for cable guidance. The method 100 comprises the following steps: In a first step 102, a longitudinal support element for supporting a cantilevering edge of the floor section in the region of the cable duct is provided. In a second step 104, the support element is temporarily displaced to provide a passageway from the cable duct to a connection opening. In a third step 106, a cable connection is guided through the connection opening.

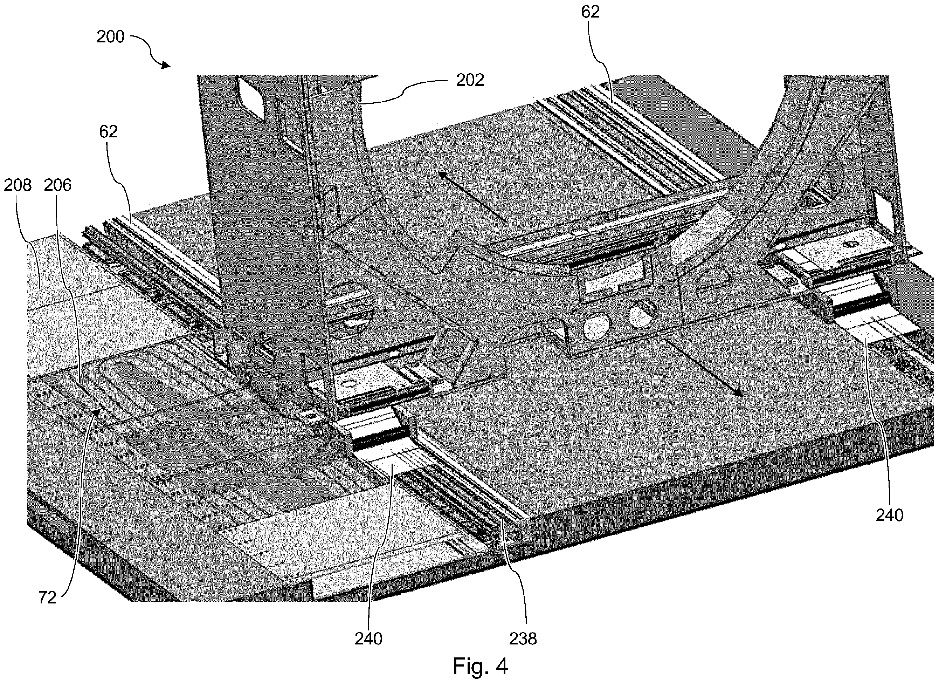

Fig. 4 shows a perspective view of a part of a movable gantry 200 of a further example of the medical system 50. The gantry 200 is part of a CT imaging system as an example for the medical appliance 52. A frame-like support structure 202 is being supported on linear bearings 204. A cable duct 206 is running along the rails on one side. The cable duct is closed by a cover arrangement 208.

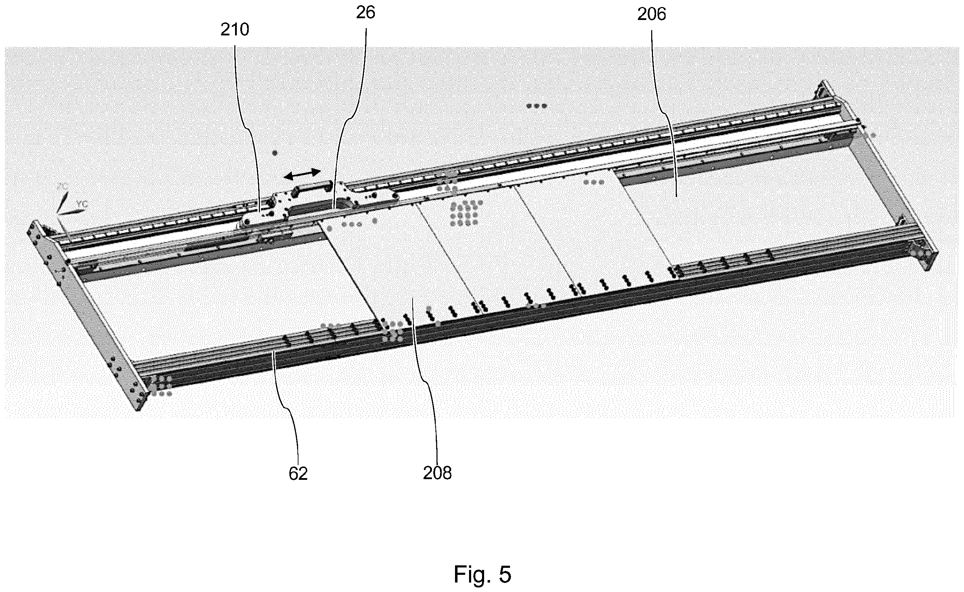

Fig. 5 shows another example of the cable guidance in a perspective view. The cable duct 206 is closed with the cover arrangement 208. A guiding bracket 210 is movable along the rails, e.g. together with the gantry 200.

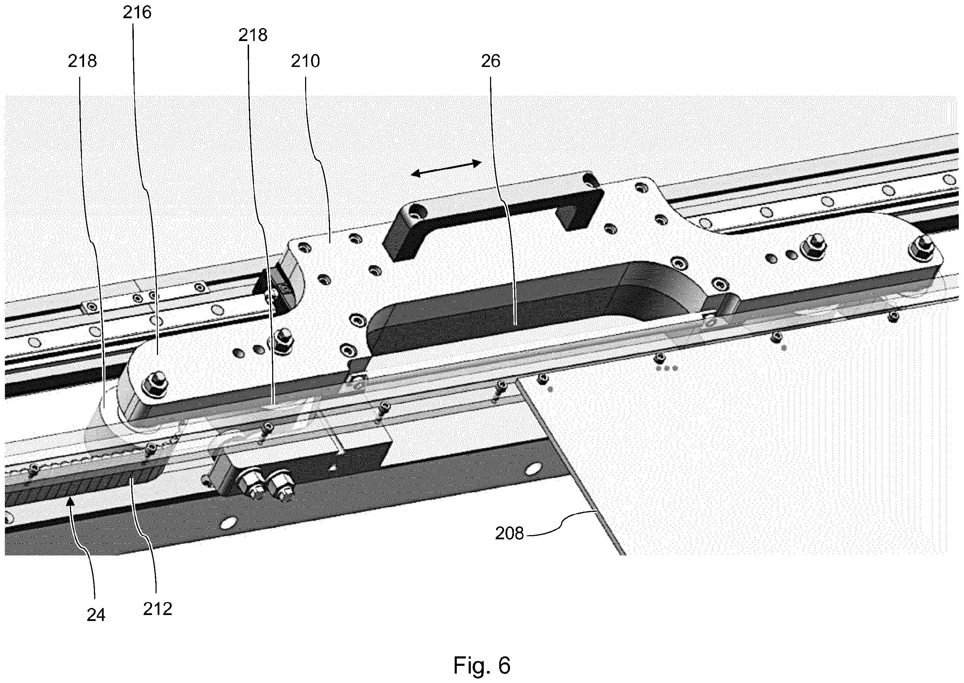

Fig. 6 shows a detail of the cable guidance of Fig. 5. The guiding bracket 210 is shown in detail together with the support element 24.

As an option, the support element 24 comprises a longitudinal flat member 212, as shown in Fig. 6, that comprises a stiffness transverse to the longitudinal direction in a plane direction of the flat member. The support element 24, i.e. the longitudinal flat member 212 in Figs. 6 to 12, is deformable in a direction transverse to the longitudinal direction and transverse to the plane direction. As an option, the support element 24, e.g. the longitudinal flat member 212, is provided as a toothed belt structure as in Figs. 6 to 12.

According to an aspect, the support element 24is provided as a longitudinal element or member that is bendable in a first direction, which is transverse to the longitudinal direction, while it is not, or at least less bendable in a second direction, which is transverse to the longitudinal direction and transverse to the first direction. Instead of a toothed belt, also other belt-like structures can be provided as long as the belt-structure is sufficiently stiff against buckling when being subject to load in the belt plane.

The term "bendable" also comprises "deformable" in the first direction transverse to the longitudinal direction. The "bending" or "transforming" is provided in order to provide the displacement along the moving direction, i.e. a successive displacement (and thus moving so-to-speak along the longitudinal direction) while moving the appliance.

In another example, not shown, the support element 24 is provided as a chain or other, longitudinal member that is bendable, or deformable like a chain.

As an option, a displacement mechanism 216 is provided that is attachable to a movable medical equipment to which cables are supplied. For example, the displacement mechanism 216 is attached to the bracket 210. The displacement mechanism 216 comprises guiding elements 218 which are configured to temporarily guide the support mechanism from a supporting position *P_{SUP}* to a non-supporting position *P_{NON},* see also Fig. 1.

The guiding elements 218 also provides that the support element 24 is held in place.

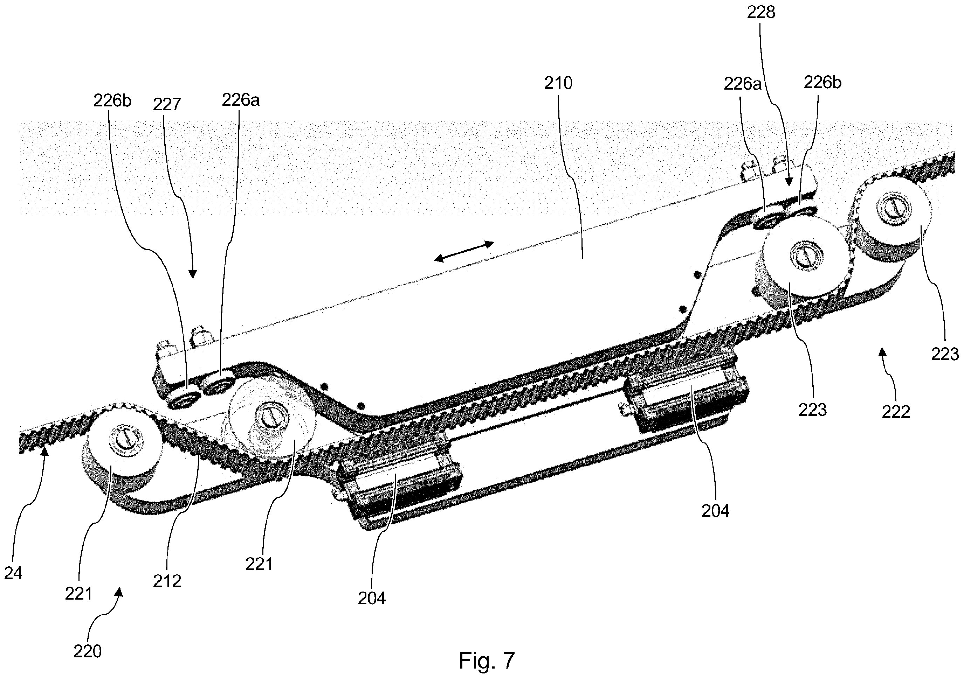

Fig. 7 shows a perspective bottom view of the detail of Fig. 6.

In an option, the guiding elements 218 comprise a first pair 220 of rotating guides 221 and a second pair 222 of rotating guides 223. The support element 24 is arranged between the rotating guides of each pair. The passageway is provided between the first pair 220 of rotating guides and the second pair 222 of rotating guides.

In an example, the rotating guides are pulleys. In another example, the rotating guides are spur gears or sprockets.

In an example, the displacement mechanism comprises a carriage that moves together with the medical appliance, like a CT-system. In an option, as shown in Fig. 7, pulleys are connected to the carriage. The moving pulleys make the gap in the moveable gap support (support belt) move together with the carriage.

The moveable gap supports (by the toothed support belt) the floor plates as a stationary support belt, covering the total stroke of the gantry. The support belt is moved to create a gap in the support. That way the cables can be led into the carriage and gantry. The support belt is moved by pulleys, connected to the gantry.

As an option, the rotating elements guiding the support element, like a belt, and the floor rollers are provided with a curved rolling surface.

As an option, the displacement mechanism 216 comprises at least one floor lifting element 226 configured to lift a part of the floor section where the support element 24 is currently being displaced.

In an example, a first floor lifting element 227 and a second floor lifting 228 element are provided. The floor lifting elements are provided to increase the height just enough to provide a release on the support element such that this can be displaced in an easier manner. The lifting reduces the friction resistance for displacing the part of the support element.

As an option, a first floor lifting element and a second floor lifting element are provided as floor rollers. As another option, the first and the second floor lifting elements are each provided as a pair of floor rollers with a lower roll 226a and an upper roll 226b. The lower roll is configured to rest on a ground surface, and the upper roll is configured to press the floor section upwards in order to release a part of the support element clamped when supporting the floor section.

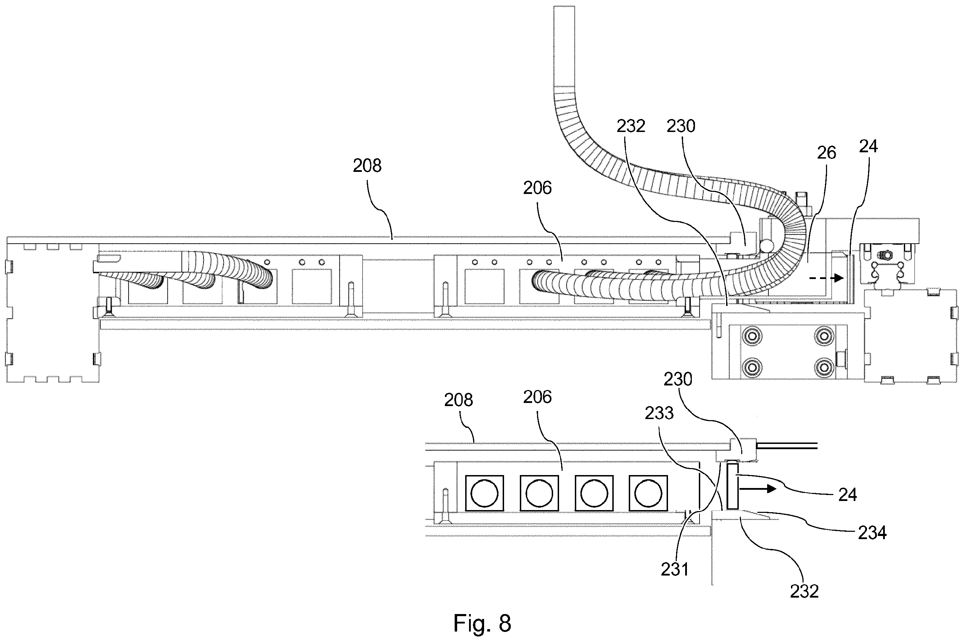

Fig. 8 shows a transverse cross-section of another example of a cable guidance. The lower part indicates the support element 24 in its supporting position *P_{SUP}.* The upper (larger) part of the drawing indicates the support element 24 in its non-supporting position *P_{NON}.* As can be seen, the cables are guided through the movable passageway 26.

As an option, the support mechanism 22 comprises a top strip 230 arranged for supporting the floor. Further, the support element 24 is provided underneath the top strip 230 for holding the top strip 230 in the supported position *P_{SUP}.*

In an option, the top strip 230 is also providing additional functions such as sealing the floor plates in a tight manner. As an example, steel plates are connected to a concrete floor on one end and to the top strip at the other end.

As an option, the support mechanism also comprises a bottom strip 232. The top strip comprises a top abutting surface 231 and the bottom strip comprises a bottom abutting surface 233. The support element 24 is clamped between the top abutting surface and the bottom abutting surface when supporting the floor. As an option, at least one of the top strip 230 and the bottom strip 232 comprises a tapered edge 234 for facilitating the insertion of the support element between the top and bottom abutting surfaces.

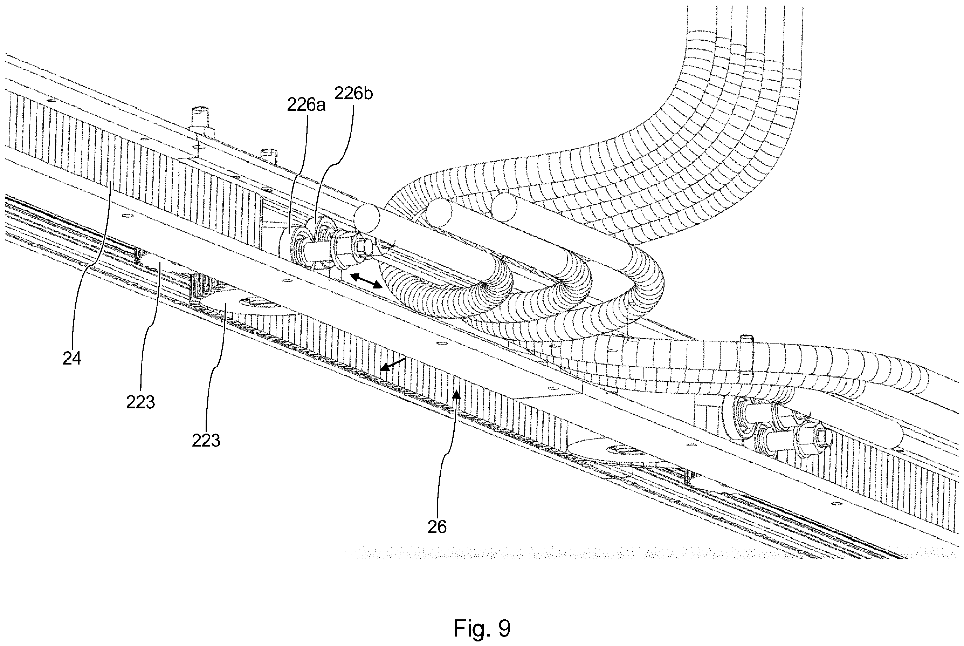

Fig. 9 shows a perspective view of the example of Fig. 8. The support element 24 in form of the longitudinal flat member is displaced in a part to allow the cables to pass through the opening.

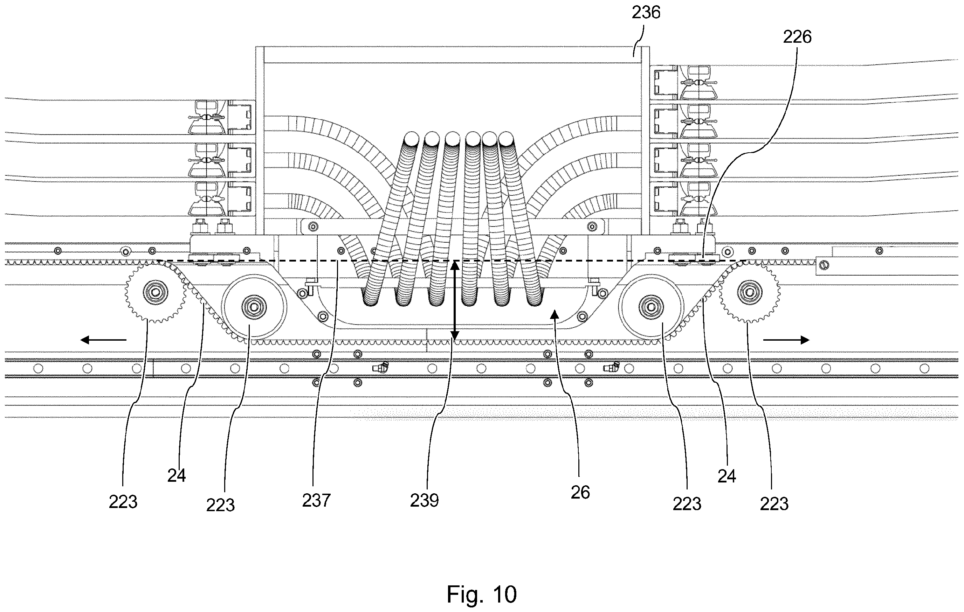

Fig. 10 shows a top view of the example of Fig. 8 and Fig. 9. When the carriage is moved, also the opening is moved by also moving the displacing of the support element 24. A cable connecting frame 236 provides stress relief for the cable connections. The cables are moved as a bundle with the cable recess.

A broken line 237 indicates the position of the supporting element 24, e.g. as the toothed belt, in its supporting position, i.e. when below the floor surface panels. A double arrow 239 indicates the displacement for achieving the free access to the below cable canal, i.e. to provide the movable passageway 26 and for achieving the supporting position along the majority of the length of the supporting element 24.

When moving the carriage of the medical appliance, and thus also the bracket 210, the rotating guides will also move and thus successively displace the support element 24. In the drawing, the bracket 210 is moved horizontally (when in landscape view of the sheet) and the toothed belt is displaced downwards to achieve a horizontal translation of the movable passageway 26. When moving to the right, for example, the toothed belt right to the most right rotating guide is about to be moved downwards. The left part of the toothed belt in the left region of the movable passageway 26 is about to be moved upwards to achieve the supporting position again.

In an example, the toothed belt is used for actively driving the medical appliance by applying a driving force to at least one of the pulleys, which for this purpose has a toothed contour matching with the toothed belt. Such driving of the appliance may be used for rough positioning, whereas further drive mechanisms may be provided for fine movement required for the imaging procedures.

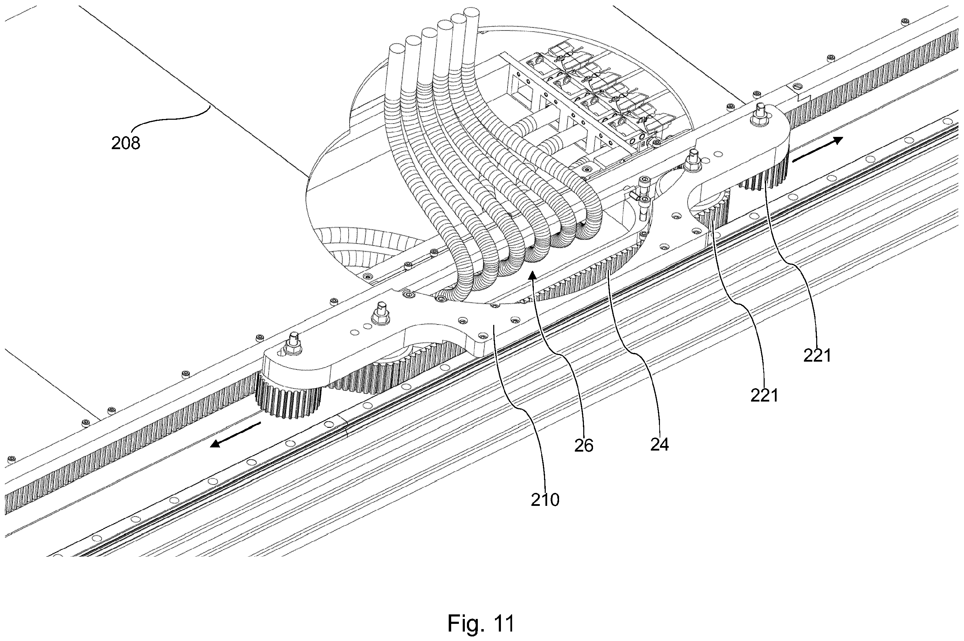

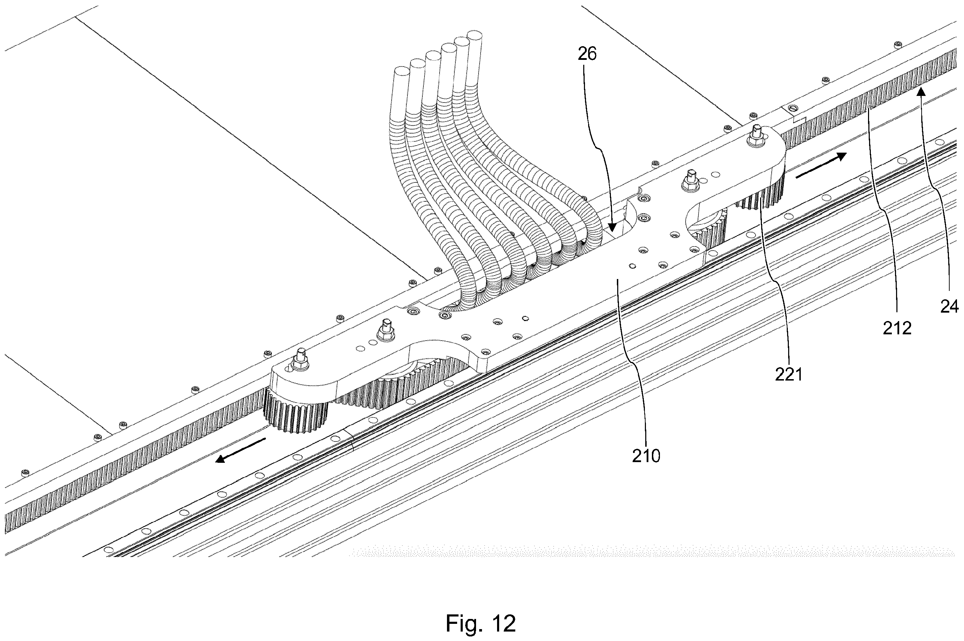

Fig. 11 shows a perspective view of the example of Fig. 10 with partly not-shown floor panels. Fig. 12 shows further perspective view of the example of Fig. 10 with intact floor panels.

In an option, see Fig. 4, the cable duct comprises a lateral part 238 that is open on an upper side. The cover arrangement 14 also comprises a longitudinal cover section 240 for temporal closing the upper side of the lateral part 238. It is also provided a lifting mechanism (not further shown in detail). The lifting mechanism is configured to temporarily displace, i.e. lift a part of the cover section such that a temporal connection opening (not visible in Fig. 4) of the cable duct 16 is provided for a feedthrough of a cable bundle.

In an example, the cable receiving and guiding space and the lateral part are for most parts separated by the support element. Where the support element is displaced, the cable receiving and guiding space and the lateral part are in communication, which allows to guide cables from the cable receiving and guiding space to the lateral part and from there via the opening to the medical appliance.

In one example, the cable duct 16 is arranged in a floor structure and the cable duct is open on an upper side towards a room. Covering elements like panels are provided to close the cable duct.

In one example, the cable duct 16 is arranged in a wall structure and the cable duct is open on a lateral side towards a room, instead of open on the upper side. Covering elements like panels are provided to close the cable duct.

As an option, it is provided that the cover section comprises a stiffness transverse to the longitudinal direction in a spanning direction across the cable duct. The cover section is deformable in a direction transverse to the longitudinal direction and transverse to the spanning direction.

In an example, the cover section is bendable like a belt, e.g. a tooth belt.

As an option, the top strip is also configured to provide a support for the cover section when temporally closing the upper side of the cable duct.

In an example, as the CT-scanner makes a linear movement, the cables exit the trough and connect to the scanner. This cable exit, or cable feedthrough, moves with the scanner along the length of the rails and floor plates. To support the floor plates on this side, a flexible belt or chain is used. This belt or chain is pushed around the cable feedthrough to create a local opening. The floor support on this position is taken over by the cable feedthrough. This way of floor support makes it possible to use thin floorplates that cover a wide trough.

As an example, the CT-scanner is moved along a pair of rails. In one example, the cable duct is provided on the side of one of the two rails. In another example, the cable duct is provided between the two rails.

In an example, the rails and flexible cable duct are placed below floor level and for this a pit is created in the structural floor. The depth of this pit is as shallow as possible to prevent weakening of the floor and to reduce costs. Over the length of the rails, the floorplates can only be supported on one side as the cable feedthrough needs to be able to pass. However, support on both sides is provided according to the present solution. Further, the flexible cable ducts are placed next to each other in a wide but shallow pit. The span that is covered by the floorplates is large but by supporting the plates on both sides, there is no need for a supporting construction or the use of thick plates. The pit depth can be kept shallow enough to avoid compromising the integrity of the structural floor. Preferably, the floor plate should be supported on both sides to maximize the available height below. The support belt provides that support but also closes the opening towards the cover.

By pushing the support belt sideways, an opening is created. The 'floor plate lift rollers' on both sides of the cable exit, push the top strip upwards to create a small local gap between support belt and top strip. As the distance between top and bottom strip is bigger than the height of the support belt at this location, the belt can be pulled-out or pushed-in without friction. The outer pulleys make sure that the belt is always placed perfectly vertical and on the correct position.

Away from the cable exit/feedthrough, the support belt is clamped between the top and bottom strip. As there are no horizontal forces present on the support belt, the belt will remain in position. There is no chance that the belt will slip and rotate. The support belt can buckle due to an excessive vertical load on the floor plate but, as the belt is made of polyurethane, this will not result is a permanent misshaped belt. Once the outer pulleys have pulled-out the belt at that point, the belt is reshaped in its original state and is fully capable of withstanding the normal load again.

In the presented design, a timing belt reinforced with Kevlar is used to meet the required buckling stiffness. A timing belt has the advantage that it will not corrode and has a limited thickness. As an alternative, a metal chain is used. The advantage of a metal chain would be the buckling stiffness.

In an example, floor loads during gantry movement can be as high as 400 kg/m2. The floor load as such can be up to 600 kg/m2. A point load of up to 150 kg can be arranged at a 70 mm wheel. The floor deflection is less than 4 mm, e.g. 2 mm at 200 kg/m2 load. Further, a pit depth of less than 80 mm is provided. The gantry stroke may be of about up to 12 m. Manual movement forces are provided up to 600 N.

As an example, the cable duct is 50 cm in width and 7 m long, or even longer, e.g. 12 m. In an example, 60 cm2 of cable cross-sectional area is provided. Motion force by feedthrough/floor support is less than 50 N.

In an example, a gantry movement of three to seven meters is provided.

The term "subject" may also be referred to as individual. The "subject" may further also be referred to as patient, although it is noted that this term does not indicate whether any illness or disease is actually present with the subject.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A cable guidance (10) with a feedthrough movable along a guide rail, the cable guidance comprising:
- a cable duct (12); and
- a cover arrangement (14);
wherein the cable duct is extending along a longitudinal direction (*D_{L})* and comprises a cable receiving and guiding space (16); and the cable duct is configured to accommodate a cable connection comprising a number of cables;
wherein the cover arrangement comprises a floor section (18) for permanently delimiting the cable duct and for forming a floor part (20);
wherein a support mechanism (22) is provided comprising a longitudinal support element (24) that is configured to support a cantilevering edge of the floor section in the region of the cable duct; and
wherein the support element is configured to be partly temporarily displaced to provide a movable passageway (26) from the cable duct to a connection opening.

2. Cable guidance according to claim 1, wherein the support element comprises a longitudinal flat member (212) that comprises a stiffness transverse to the longitudinal direction in a plane direction of the flat member
wherein the support element is deformable in a direction transverse to the longitudinal direction and transverse to the plane direction; and
wherein the support element is provided as a toothed belt structure.

3. Cable guidance according to claim 1 or 2, wherein a displacement mechanism (216) is provided that is attachable to a movable medical equipment to which cables are supplied; and
wherein the displacement mechanism comprises guiding elements (218) which are configured to temporarily guide the support mechanism from a supporting position (*P_{SUP}*) to a non-supporting position (*P_{NON}*)*.*

4. Cable guidance according to claim 3, wherein the guiding elements comprise a first pair (220) of rotating guides and a second pair (222) of rotating guides; and the support element is arranged between the rotating guides of each pair; and
wherein the passageway is provided between the first pair of rotating guides and the second pair of rotating guides.

5. Cable guidance according to one of the preceding claims, wherein the support mechanism comprises a top strip (230) arranged for supporting the floor; and
wherein the support element is provided underneath the top strip for holding the top strip.

6. Cable guidance according to claim 5, wherein the support mechanism also comprises a bottom strip (232);
wherein the top strip comprises a top abutting surface (231) and the bottom strip comprises a bottom abutting surface (233); and wherein the support element is clamped between the top abutting surface and the bottom abutting surface when supporting the floor; and
wherein at least one of the top strip and the bottom strip comprises a tapered edge (234) for facilitating the insertion of the support element between the top and bottom abutting surfaces.

7. Cable guidance according to one of the preceding claims, wherein the displacement mechanism comprises at least one floor lifting element (226) configured to lift a part of the floor section where the support element is currently being displaced.

8. Cable guidance according to claim 7, wherein a first floor lifting element (227) and a second floor lifting element (228) are provided as floor rollers;
wherein the first and the second floor lifting elements are each provided as a pair of floor rollers with a lower roll (226a) and an upper roll (226b); and
wherein the lower roll is configured to rest on a ground surface, and the upper roll is configured to press the floor section upwards in order to release a part of the support element clamped when supporting the floor section.

9. Cable guidance according to one of the preceding claims, wherein the cable duct comprises a lateral part (238) that is open on an upper side; and the cover arrangement also comprises a longitudinal cover section (240) for temporal closing the upper side of the lateral part;
wherein it is also provided a lifting mechanism; and
wherein the lifting mechanism is configured to temporarily lift a part of the cover section such that a temporal connection opening of the cable duct is provided for a feedthrough of a cable bundle.

10. Cable guidance according to claim 9, wherein the cover section comprises a stiffness transverse to the longitudinal direction in a spanning direction across the cable duct; and
wherein the cover section is deformable in a direction transverse to the longitudinal direction and transverse to the spanning direction.

11. Cable guidance according to claim 9 or 10, wherein the top strip is also configured to provide a support for the cover section when temporally closing the upper side of the cable duct.

12. A medical system (50) comprising:
- a medical appliance (52);
- a movable support (54);
- a cable supply (56) for a cable connection of the medical appliance; and
- a cable guidance (10) according to one of the preceding claims;
wherein the cable supply comprises a plurality of cables; and
wherein the medical appliance is movably supported by the movable support;
wherein the plurality of cables is arranged within the cable duct of the cable guidance and extending through the passageway and the connection opening to the medical appliance.

13. System according to claim 12, wherein the movable support comprises at least one rail track (62) with at least one linear bearing (204).

14. System according to claim 12 or 13, wherein the medical appliance is a CT scanner

15. A method (100) for cable guidance, comprising the following steps:
- providing (102) a longitudinal support element for supporting a cantilevering edge of the floor section in the region of the cable duct;
- temporarily displacing (104) the support element to provide a passageway from the cable duct to a connection opening; and
- guiding (106) a cable connection through the connection opening.
